# FASCICULE DE BREVET EUROPEEN

(11) **EP 1 781 757 B1**
(45) Date de publication et mention de la délivrance du brevet: **23.07.2008**
(21) Numéro de dépôt: 05797578.1
(22) Date de dépôt: 08.08.2005
(51) Int. Cl.: C09K 11/77

(54) **MATERIAU SCINTILLATEUR DENSE ET RAPIDE A FAIBLE LUMINESCENCE RETARDEE**
DICHTES UND SCHNELLES SZINTILLATORMATERIAL MIT LEICHT VERZÖGERTER LUMINESZENZ
LOW-DELAYED LUMINESCENCE DENSE AND RAPID SCINTILLATOR MATERIAL

(30) Priorité: 09.08.2004 FR 0451815
(43) Date de publication de la demande: 09.05.2007
(73) Titulaire: Saint-Gobain Cristaux & Detecteurs, 92400 Courbevoie (FR)
(72) Inventeur: FERRAND, Bernard, F-38340 VOREPPE (FR); VIANA, Bruno, F-91230 MONTGERON (FR); PIDOL, Ludivine, F-69003 LYON (FR); DORENBOS, Pieter, NL-2281 GM RIJSWIJK (NL)
(74) Mandataire: Colombier, Christian
(86) Numéro de dépôt international: PCT/FR2005/050658
(87) Numéro de publication internationale: WO 2006/018586

(56) Documents cités:
- US-B1- 6 323 489
- US-B1- 6 624 420
- ZAVARTSEV YU D ET AL: "Czochralski growth and characterisation of large Ce<3+>:Lu2SiO5 single crystals co-doped with Mg<2+> or Ca<2+> or Tb<3+> for scintillators" JOURNAL OF CRYSTAL GROWTH, ELSEVIER, AMSTERDAM, NL, vol. 275, no. 1-2, 15 février 2005 (2005-02-15), pages e2167-e2171, XP004823565 ISSN: 0022-0248

## Description

L'invention concerne des matériaux scintillateurs, un procédé de fabrication permettant de les obtenir et l'utilisation desdits matériaux, notamment dans des détecteurs pour rayons gamma et/ou rayons X.

Les matériaux scintillateurs sont largement utilisés dans des détecteurs pour rayons gamma, rayons X, rayons cosmiques et particules dont l'énergie est de l'ordre de 1 KeV et également supérieure à cette valeur.

Un matériau scintillateur est un matériau transparent dans le domaine de longueur d'onde de scintillation, qui répond à un rayonnement incident par l'émission d'une impulsion lumineuse.

A partir de tels matériaux, généralement des monocristaux, on peut fabriquer des détecteurs où la lumière émise par le cristal que comprend le détecteur, est couplée à un moyen de détection de la lumière et produit un signal électrique proportionnel au nombre d'impulsions lumineuses reçus et à leur intensité. De tels détecteurs sont utilisés notamment dans l'industrie pour des mesures d'épaisseur ou de grammage, dans les domaines de la médecine nucléaire, de la physique, de la chimie, de la recherche pétrolière.

Une famille de cristaux scintillateurs connus et utilisés est celle des silicates de lutécium dopés au cérium. Lu₂SiO₅ dopé au cérium est décrit dans US 4,958,080. Le brevet US 6,624,420 décrit Ce₂ₓ(Lu_{1-y}Y_{y})₂₍₁₋ₓ₎SiO₅. Enfin, US 6,437,336 porte sur les compositions du type Lu₂₍₁₋ₓ₎M₂ₓSi₂O₇, où M est au moins partiellement du cérium. Ces diverses compositions de scintillateurs ont toutes en commun de posséder un fort pouvoir d'arrêt des rayonnements de haute énergie, de donner lieu à une émission de lumière intense avec des impulsions lumineuses très rapides.

Une propriété additionnelle souhaitable est de réduire la quantité de lumière émise après arrêt du rayonnement incident, (ou luminescence retardée « afterglow » en anglais). Physiquement, ce phénomène bien connu de l'homme de l'art est expliqué par la présence de pièges électroniques dans la structure cristallographique du matériau. Le phénomène de scintillation repose sur l'effet photo-électrique, qui donne lieu dans le matériau scintillateur à la création d'une paire électron-trou. L'électron en se recombinant sur un site actif (dans les scintillateurs précités, un site Ce³⁺) donne lieu à l'émission de photons, selon un processus généralement bien inférieur à la microseconde. Les scintillateurs précités, particulièrement rapides, donnent lieu à une durée d'impulsion qui décroît avec une constante exponentielle de premier ordre de l'ordre de 40 ns. Les électrons piégés en revanche ne génèrent pas de lumière, mais leur dépiégeage par excitation thermique (y compris à température ambiante) donne lieu à une émission de photons, l'afterglow, qui reste encore mesurable sur des temps supérieurs à la seconde.

Ce phénomène peut être rédhibitoire dans les applications où l'on cherche à isoler chaque impulsion par des fenêtrages très courts. C'est en particulier le cas des applications de Computed Tomography (scanners « CT »), bien connus dans les secteurs médicaux ou industriels. Lorsque le système CT est couplé à un scanner TEP (Tomographie à Emission de Positons), ce qui devient la norme dans l'industrie, la dégradation de la résolution du CT affecte la performance de l'ensemble du système et donc la capacité du clinicien à interpréter le résultat du système complet TEP/CT. L'afterglow est connu comme étant très rédhibitoire pour ces applications.

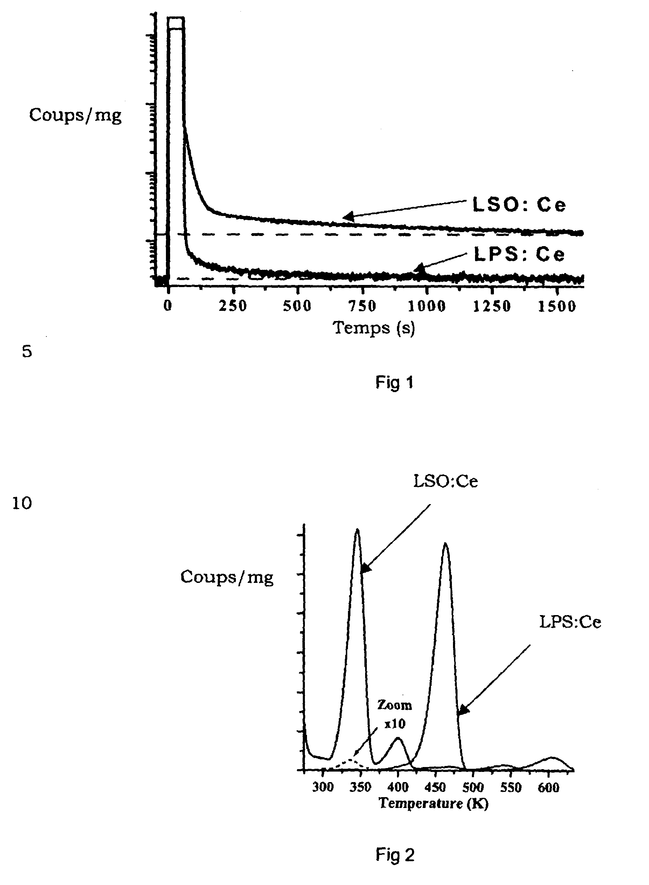

Les compositions de type silicates de lutécium citées dans US 4,958,080 (de type « LSO : Ce », selon les notations de l'homme de l'art) et US 6,624,420 (de type « LYSO : Ce ») sont connues pour générer un afterglow significatif. Les compositions décrites dans US 6,437,336 (de type « LPS : Ce ») ont en revanche l'avantage d'un afterglow beaucoup moins important. Ces résultats sont donnés par exemple dans : L. Pidol, A. Kahn-Harari, B. Viana, B. Ferrand, P. Dorenbos, J. de Haas, C.W.E. van Eijk and E. Virey, "Scintillation properties of Lu2Si2O7 : Ce3+, a fast and dense scintillator crystal", Journal of Physics : Condensed Matter, 2003, 15, 2091-2102. La courbe de la figure 1 en est extraite et représente la quantité de lumière détectée sous forme de nombre d'événements (ou coups) par mg de matériau scintillateur, en fonction du temps, sous excitation X pendant quelques heures. La composition « LPS : Ce» donne un résultat significativement meilleur en afterglow.

Le comportement du LYSO est très proche de celui du LSO de ce point de vue. La limitation de cet afterglow fait l'objet de la présente demande.

La propriété d'afterglow peut être mise en évidence de façon plus fondamentale par thermoluminescence (voir S.W.S. McKeever « Thermoluminescence of solids », Cambridge University Press (1985)). Cette caractérisation consiste à exciter thermiquement un échantillon après irradiation et à mesurer l'émission lumineuse. Un pic lumineux proche de la température ambiante à 300 K traduit un afterglow plus ou moins important selon son intensité (dépiégeage). Un pic à température plus élevée traduit l'existence de pièges plus profonds moins susceptibles d'excitation thermique à température ambiante. Ceci est illustré dans la figure 2, extraite de l'article de L. Pidol et al précité, qui montre autrement l'avantage d'une composition de type LPS en termes d'afterglow.

Cependant, les compositions de type LPS présentent l'inconvénient d'un pouvoir d'arrêt moindre que celles de type LSO ou LYSO. Cet état de fait découle simplement du numéro atomique moyen du composé et de la densité de la phase associée.

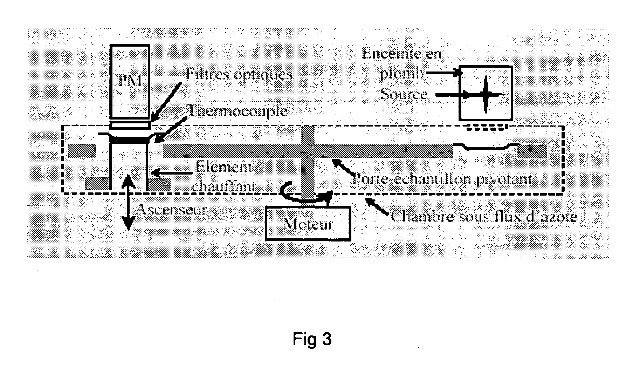

On peut réaliser les mesures de thermoluminescence avec un appareil automatisé TL-DA-15, fabriqué par RISO (Danemark), schématisé sur la figure 3. L'élément chauffant, le thermocouple et un "ascenseur", permettant de positionner l'échantillon, se situent dans l'alignement du photomultiplicateur (PM) et de filtres optiques. A l'intérieur de la chambre d'analyse sous flux d'azote, une table pivotante (porte échantillon pivotant) actionnée par un moteur peut positionner l'échantillon soit au niveau de la source radioactive (placée dans une enceinte en plomb) pour l'étape d'irradiation, soit entre l'élément chauffant et le photomultiplicateur pour les mesures de thermoluminescence. Avant chaque mesure, les cristaux, d'environ 1 mm d'épaisseur, sont chauffés quelques minutes à 672 K. Ils sont ensuite irradiés puis les courbes de thermoluminescence sont enregistrées sous flux d'azote, avec une vitesse de chauffage constante, entre 313 et 672 K. Les mesures à plus haute température sont rendues impossible par la radiation du corps noir (la lumière spontanément émise par une substance qui est chauffée de façon incandescente est appelée radiation du corps noir). Chaque courbe est normalisée par rapport à la masse de produit.

Dans notre cas, l'émission qui nous intéresse est celle de l'ion cérium, entre 350 et 450 nm environ. Nous avons choisi des filtres adaptés (HA3 et 7-59) en entrée du photomultiplicateur. Pour des mesures quantitatives, l'irradiation se fait in-situ par une source β⁹⁰Sr/⁹⁰Y, fournissant une dose de 3,6 Gray/h dans l'air. Les paramètres variables lors des mesures de TL (thermoluminescence) sont la dose (temps d'irradiation, 20 s ici) et la vitesse de chauffage (0,5 K/s ici).

La demanderesse a découvert que l'ajout d'un alcalino-terreux bivalent M et/ou d'un métal trivalent M' à une composition de type LYSO permettait de réduire la luminescence retardée de façon très importante. Notamment, M peut être Ca, Mg ou Sr (sous forme de cation bivalent). Notamment, M' peut être Al, Ga ou In (sous forme de cation trivalent). L'élément M vient en substitution de Y ou Lu et l'élément M' vient en substitution de Si.

Les produits selon l'invention, grâce à l'introduction de M, notamment Ca, permettent de manière surprenante de réduire l'afterglow, sans affecter la densité dans les proportions considérées.

Le matériau scintillateur selon l'invention est de formule

Lu_{(2-y)}Y_{(y-z-x)}CeₓM_{z}Si₍₁₋ᵥ₎M'ᵥO₅ (formule I)

dans laquelle
M représente un alcalino-terreux bivalent comme Ca, Mg ou Sr et M' représente un métal trivalent comme Al, Ga ou In,
(z+v) étant supérieur ou égal à 0,0001 et inférieur ou égal à 0,2 ,
z étant supérieur ou égal à 0 et inférieur ou égal à 0,2,
v étant supérieur ou égal à 0 et inférieur ou égal à 0,2 ,
x étant supérieur ou égal à 0,0001 et inférieur à 0,1
y allant de (x+z) à 1,

De préférence, (z+v) est supérieur ou égal à 0,0002

De préférence, (z+v) est inférieur ou égal à 0,05 et de manière encore préférée inférieur ou égale à 0,01, et même inférieur à 0,001.

De préférence, x est supérieur à 0,0001 et inférieur à 0,001.

Notamment, v peut être nul (absence de M'), auquel cas z est d'au moins 0,0001.

Notamment, le matériau scintillateur selon l'invention peut être tel que v soit nul. Egalement, le matériau scintillateur selon l'invention peut être tel que M soit Ca, ce qui correspond à une composition particulièrement adaptée. La combinaison de v étant nul avec M étant Ca est particulièrement adaptée. La composition selon l'invention a alors la formule suivante :

Lu_{(2-y})Y_{(y-z-x)}CeₓCa_{z}SiO₅ (formule II)

Egalement, le matériau scintillateur selon l'invention peut notamment être tel que z soit nul. Egalement, le matériau scintillateur selon l'invention peut notamment être tel que M' soit Al. La combinaison de z étant nul avec M' étant Al est particulièrement adaptée. La composition selon l'invention a alors la formule suivante :

Lu_{(2-y)}Y_{(y-x)}CeₓAlᵥSi₍₁₋ᵥ₎O₅, (formule III)

La teneur molaire en élément O est de sensiblement 5 fois celle en (Si + M'), étant entendu que cette valeur peut varier de l'ordre de + ou - 2%.

Le matériau scintillateur selon l'invention peut être obtenu sous forme de monocristal par croissance du type Czochralski.

L'invention concerne également l'utilisation du matériau scintillateur selon l'invention comme composant d'un détecteur de rayonnement notamment par rayons gamma et/ou rayons X, notamment dans les scanners de Computed Tomography (CT).

L'invention concerne également l'utilisation du matériau scintillateur selon l'invention comme composant d'un détecteur de scintillation notamment pour des applications dans l'industrie, le domaine médical et/ou la détection pour le forage pétrolier. Est notamment concerné tout système scintillateur avec acquisition en continu (ce qui inclut la tomographie CT, c'est-à-dire la « computed tomographie » en anglais). Est également concerné tout système scintillateur du type tomographie par émission de positons notamment avec temps de vol (« time of flight » en anglais), le cas échéant combiné avec de la tomographie d'émission.

Sans que la demanderesse ne soit tenue par aucun argument théorique, il est supposé que l'introduction d'un ion bivalent alcalino-terreux M en substitution d'un ion trivalent de terre rare, ou d'un ion trivalent métallique M' en substitution d'un atome tétravalent de silicium, crée un déficit de charge positive qui limite le piégeage des électrons responsables de l'afterglow.

### EXEMPLES

On a réalisé selon le procédé Czochralski dans des conditions identiques décrites dans les brevets précités trois monocristaux de LYSO:Ce de 1 pouce de diamètre. Pour ce faire, on a utilisé des matières premières correspondant à ces compositions :
Référence (sans Ca) :
   Lu_{1,8}Y_{0,1978}Ce_{0,0022}SiO₄,₉₉₆₁
Composition 1 :
   Lu_{1,8}Y_{0,1778}Ca_{0,02}Ce_{0,0022}SiO₄,₉₉₆₁
Composition 2 :
   Lu_{1,8}Y_{0,1878}Ca_{0,01}Ce_{0,0022}SiO₄,₉₉₆₁

Les charges sont préparées à partir des oxydes correspondant (de Ca, Ce, Lu, Y) de manière à obtenir les formules souhaitées. Les concentrations réelles dans le cristal final en Ce et Ca sont plus faibles que celles introduites par les matières premières par ségrégation lors de la cristallogénèse.

Les monocristaux finalement obtenus, de formule
Lu_{(2-y)}Y_{(y-z-x)}CeₓCa_{z}SiO₅, ont les compositions suivantes en tête d'échantillon :

| | Référence (sans Ca) | Composition 1 | Composition 2 |
|---|---|---|---|
| x | 0,00026 | 0,00031 | 0,00036 |
| y | 0,095 | 0,095 | 0,095 |
| z | 0 | 0,00041 | 0,00023 |

La composition 1 donne un afterglow significativement moindre que celui de la composition de référence (du type classique LYSO) et un niveau lumineux estimé à 20 000 photons / Mev sous excitation par une source de rayons gamma ¹³⁷Cs, soit légèrement moins que les compositions LPS (26 000 photons / MeV), LYSO (34 000 photons / MeV) et LSO (environ 28 000 photons / MeV). Un tel niveau lumineux n'est absolument pas rédhibitoire pour la plupart des applications. Le germanate de bismuth (Bi₄Ge₃O₁₂), très couramment utilisé, n'émet que 9000 photons / MeV. Au total, la composition 1 permet de conserver le pouvoir d'arrêt d'une composition de type LYSO, sans perdre significativement en niveau lumineux, tout en diminuant significativement l'afterglow.

La composition 2 est encore plus intéressante avec un afterglow encore bien inférieur et un rendement lumineux de 27 000 photons / MeV.

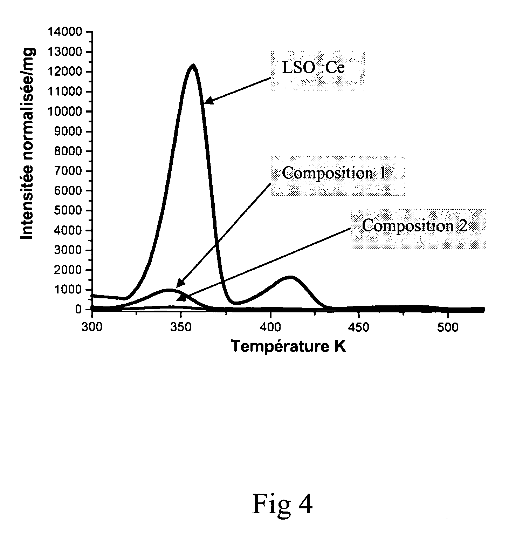

La figure 4 compare les afterglow des compositions 1 et 2 avec un LSO classique (référence).

## Revendications

1. Matériau scintillateur inorganique de formule
Lu_{(2-y)}Y_{(y-z-x)}Ceₓ(M_{z}Si₍₁₋ᵥ₎M'ᵥO₅,
dans laquelle
M représente un ion alcalino-terreux bivalent et M' représente un métal trivalent,
(z+v) étant supérieur ou égal à 0,0001 et inférieur ou égal à 0,2
z étant supérieur ou égal à 0 et inférieur ou égal à 0,2 ,
v étant supérieur ou égal à 0 et inférieur ou égal à 0,2 ,
x étant supérieur ou égal à 0,0001 et inférieur à 0,1
y allant de (x+z) à 1 .

2. Matériau selon la revendication précédente, **caractérisé en ce que** (z+v) est supérieur ou égal à 0,0002

3. Matériau selon l'une des revendications précédentes, **caractérisé en ce que** (z+v) est inférieur ou égal à 0,05.

4. Matériau selon la revendication précédente, **caractérisé en ce que** (z+v) est inférieur ou égal à 0,01 .

5. Matériau selon la revendication précédente, **caractérisé en ce que** (z+v) est inférieur ou égal à 0,001.

6. Matériau selon l'une des revendications précédentes, **caractérisé en ce que** x est supérieur à 0,0001 et inférieur à 0,001.

7. Matériau selon l'une des revendications précédentes, **caractérisé en ce que** M est choisi parmi Ca, Mg et Sr.

8. Matériau selon l'une des revendications précédentes, **caractérisé en ce que** M' est choisi parmi Al, In, Ga.

9. Matériau selon l'une des revendications précédentes, **caractérisé en ce que** v est nul.

10. Matériau selon l'une des revendications précédentes, **caractérisé en ce que** M est Ca.

11. Matériau selon l'une des revendications 1 à 8, **caractérisé en ce que** z est nul.

12. Matériau selon l'une des revendications précédentes, **caractérisé en ce que** M' est Al.

13. Matériau scintillateur selon l'une des revendications précédentes, **caractérisé en ce qu'**il est monocristallin.

14. Procédé de croissance du matériau scintillateur monocristallin de la revendication précédente, **caractérisé en ce qu'**il est obtenu par le procédé Czochralski.

15. Détecteur de scintillation comprenant un matériau scintillateur inorganique de l'une des revendications 1 à 13.

16. Scanner CT (Computed Tomography) comprenant un détecteur de la revendication précédente.

17. Utilisation du matériau scintillateur de l'une des revendications 1 à 13 comme composant d'un détecteur de scintillation notamment pour des applications dans l'industrie, le domaine médical et/ou la détection pour le forage pétrolier

18. Utilisation du matériau scintillateur de l'une des revendications 1 à 13 comme élément d'un scanner de Computed Tomography (CT) et/ou élément de scanner du type tomographie par émission de positons, notamment avec temps de vol.

## Claims

1. An inorganic scintillator material of formula
Lu_{(2-y)}Y_{(y-z-x)}CeₓM_{z}Si₍₁₋ᵥ₎M'ᵥO₅,
in which:
M represents a divalent alkaline earth ion and M' represents a trivalent metal,
(z+v) being greater than or equal to 0.0001 and less than or equal to 0.2;
z being greater than or equal to 0 and less than or equal to 0.2;
v being greater than or equal to 0 and less than or equal to 0.2;
x being greater than or equal to 0.0001 and less than 0.1; and
y ranging from (x+z) to 1.

2. The material as claimed in the preceding claim, **characterized in that** (z+v) is greater than or equal to 0.0002.

3. The material as claimed in either of the preceding claims, **characterized in that** (z+v) is less than or equal to 0.05.

4. The material as claimed in the preceding claim, **characterized in that** (z+v) is less than or equal to 0.01.

5. The material as claimed in the preceding claim, **characterized in that** (z+v) is less than or equal to 0.001.

6. The material as claimed in one of the preceding claims, **characterized in that** x is greater than 0.0001 and less than 0.001.

7. The material as claimed in one of the preceding claims, **characterized in that** M is chosen from Ca, Mg and Sr.

8. The material as claimed in one of the preceding claims, **characterized in that** M' is chosen from Al, In and Ga.

9. The material as claimed in one of the preceding claims, **characterized in that** v is zero.

10. The material as claimed in one of the preceding claims, **characterized in that** M is Ca.

11. The material as claimed in one of claims 1 to 8, **characterized in that** z is zero.

12. The material as claimed in one of the preceding claims, **characterized in that** M' is Al.

13. The scintillator material as claimed in one of the preceding claims, **characterized in that** it is a single-crystal material.

14. A process for growing the single-crystal scintillator material of the preceding claim, **characterized in that** it is obtained by the Czochralski method.

15. A scintillation detector comprising an inorganic scintillator material of one of claims 1 to 13.

16. A CT (Computed Tomography) scanner comprising a detector of the preceding claim.

17. Use of the scintillator material of one of the claims 1 to 13 as a component of a scintillation detector, especially for applications in industry, for the medical field and/or for detection in oil drilling.

18. The use of the scintillator material as claimed in one of the claims 1 to 13 as an element of a CT scanner and/or an element of a PET (positron emission tomography) scanner, especially a time-of-flight PET scanner.

## Patentansprüche

1. Anorganisches Szintillatormaterial mit der Formel
Lu_{(2-y)}Y_{(y-z-x)}CeₓM_{z}Si₍₁₋ᵥ₎M'ᵥO₅,
in welcher
M ein zweiwertiges Erdalkalimetallion und M' ein dreiwertiges Metall bedeutet und
(z+v) größer als oder gleich 0,0001 und kleiner als oder gleich 0,2,
z größer als oder gleich 0 und kleiner als oder gleich 0,2,
v größer als oder gleich 0 und kleiner als oder gleich 0,2,
x größer als oder gleich 0,0001 und kleiner als 0,1 und
y (x+z) bis 1 ist.

2. Material nach dem vorhergehenden Anspruch, **dadurch gekennzeichnet, dass** (z+v) größer als oder gleich 0,0002 ist.

3. Material nach einem der vorhergehenden Ansprüche, **dadurch gekennzeichnet, dass** (z+v) kleiner als oder gleich 0,05 ist.

4. Material nach dem vorhergehenden Anspruch, **dadurch gekennzeichnet, dass** (z+v) kleiner als oder gleich 0,01 ist.

5. Material nach dem vorhergehenden Anspruch, **dadurch gekennzeichnet, dass** (z+v) kleiner als oder gleich 0,001 ist.

6. Material nach einem der vorhergehenden Ansprüche, **dadurch gekennzeichnet, dass** x größer als 0,0001 und kleiner als 0,001 ist.

7. Material nach einem der vorhergehenden Ansprüche, **dadurch gekennzeichnet, dass** M aus Ca, Mg und Sr ausgewählt ist.

8. Material nach einem der vorhergehenden Ansprüche, **dadurch gekennzeichnet, dass** M' aus Al, In und Ga ausgewählt ist.

9. Material nach einem der vorhergehenden Ansprüche, **dadurch gekennzeichnet, dass** v gleich Null ist.

10. Material nach einem der vorhergehenden Ansprüche, **dadurch gekennzeichnet, dass** M Ca bedeutet.

11. Material nach einem der Ansprüche 1 bis 8, **dadurch gekennzeichnet, dass** z gleich Null ist.

12. Material nach einem der vorhergehenden Ansprüche, **dadurch gekennzeichnet, dass** M' Al bedeutet.

13. Szintillatormaterial nach einem der vorhergehenden Ansprüche, **dadurch gekennzeichnet, dass** es monokristallin ist.

14. Verfahren zum Züchten des monokristallinen Szintillatormaterials nach dem vorhergehenden Anspruch, **dadurch gekennzeichnet, dass** es durch das Czochralski-Verfahren erhalten wird.

15. Szintillationsdetektor, der ein anorganisches Szintillatormaterial nach einem der Ansprüche 1 bis 13 umfasst.

16. CT-(Computertomographie-)Scanner, der einen Detektor nach dem vorhergehenden Anspruch umfasst.

17. Verwendung des Szintillatormaterials nach einem der Ansprüche 1 bis 13 als Komponente eines Szintillationsdetektors, insbesondere für Verwendungen in der Industrie, auf dem Gebiet der Medizin und/oder der Detektion für die Erdölbohrung.

18. Verwendung des Szintillatormaterials nach einem der Ansprüche 1 bis 13 als Bestandteil eines Computertomographie-(CT-)Scanners und/oder Scannerbestandteil der Positronenemissionstomographie, insbesondere mit Flugzeit.
